# EUROPEAN PATENT APPLICATION

(11) **EP 2 014 178 A1**
(43) Date of publication of application: **14.01.2009**
(21) Application number: 08167768.4
(22) Date of filing: 23.12.2005
(51) Int. Cl.: A23L 1/29, A23L 1/308, A23L 1/30, A23L 1/304, A61P 1/00, A61P 25/02, A61P 17/02

(54) **Temporally meal menu for infants**

(30) Priority: 23.12.2004 EP 04078500
(62) Divisional of application: 05824607.5
(71) Applicant: Nutricia N.V., 2712 HM Zoetermeer (NL)
(72) Inventor: Bijkerk, Elise, 3031 ED, Rotterdam (NL); Alles, Martine Sandra, 7328 NH, Apeldoorn (NL)
(74) Representative: Swinkels, Bart Willem

(57) **Abstract**

The invention concerns a kit of parts and a method for providing nutrition which provides within a time period of 5 to 10 days a nutritional composition comprising 0.35 to 6 mg iron per 100 g, a nutritional composition comprising 1 to 10 g fibre per 100 g and a nutritional composition comprising 10 to 5000 mg long chain poly unsaturated fatty acids per 100 g to an infant.

## Description

### FIELD OF THE INVENTION

The invention relates to a method for providing nutrition to infants.

### BACKGROUND OF THE INVENTION

Between the age of 4 to 36 months, children are gradually introduced to the adult diet. In this transition period, the diet changes from breast milk, infant formulae and/or follow on formulae to solid and semi-solid foods, unadapted cow's milk and other beverages. During this period the child increasingly becomes accustomed to eating adult-type foods and becomes familiar with a wide range of textures and tastes.

Providing a nutritionally well-balanced diet is in this group very important, since nutrition in the early years of life is a major determinant of healthy growth and development throughout childhood and of good health in adulthood. In this particular phase of development, the infant has specific nutritional requirements.

Offering home cooked dinners does not always result in this well-balanced diet on a weekly basis, since often in a household one is not accustomed to cooking a great variety of meals, for instance because of habit, limited knowledge on nutrition or time pressure. This often results in certain deficiencies occurring, possibly resulting in a sub-optimal development or even impaired health. In an attempt to prevent deficiencies from occurring, infants are sometimes overfed. Overfeeding may have significant adverse effects. Therefore, a need exists for nutritionally well-balanced diet for infants, which provides on a weekly basis, an adequate intake of essential nutrients.

### SUMMARY OF THE INVENTION

The present invention provides a method for providing a balanced diet to infants. The present inventors found that especially iron, long chain polyunsaturated fatty acids (LC-PUFA) and dietary fibre are insufficiently ingested by many infants. Also insufficient amounts of zinc are usually ingested.

Iron requirements in infants and young children are high, due to the fast growth and development. Low iron levels may result in iron deficiency anaemia and have an impact on cognitive skills, when body iron stores are depleted. Many infants above 1 year of age consume a lot of unadapted cow's milk, and this results in a low overall intake of iron, since unadapted cow's milk decreases the bioavailability of iron.

Dietary fibre is beneficial for infants because it normalises the defecation pattern and consistency. The intake of dietary fibre has a beneficial effect in constipation, prevents infection and/or stimulates the immune system through stimulating the colonic flora. Fibres have a saturating effect, and hence have a beneficial effect on satiety and sleep.

Long chain poly unsaturated fatty acids (LC-PUFA) are essential components of the brain, eyes and nervous system. Particular during a child's first years LC-PUFA have an important nutritional benefit in that they are important for mental and neurological functions. The role of omega-3 LC-PUFA fatty acids in brain development makes their dietary intake particularly important for infants. Low intake of LC-PUFA intake has been linked with several health issues, including attention deficit hyperactivity disorder (ADHD), digestive problems, eczema, asthma and multiple allergies. Also intake of sufficient amounts of LC-PUFA in an infant can prevent diseases occurring later in life, such as hypertension.

Zinc is essential for all growth processes, including those of the brain and hence the requirement of zinc is increased in infants compared to adults. Zinc is further important for a healthy immune system and for healing wounds. Mild cases of zinc deficiency in humans include slight weight loss and hyperammonaemia. As a result of its deficiency, growth is affected adversely.

Generally, it is recommended that the daily food intake be divided amongst three meals a day and 2 up to 3 snack moments. However, a substantial part of the diet, on a caloric basis, is derived from the dinner, the main meal of the day. In infants the caloric intake of this ranges from 15 to 35% of the daily caloric intake. So having, on a weekly basis, nutritionally well-balanced dinners significantly contributes to the health of infants.

The present nutritionally well-balanced nutrition on a weekly basis contains adequate amounts of iron, LC-PUFA, dietary fibre and optionally zinc. The present menu provides the adequate amounts over a prolonged time period, because administration of high dosages of these nutrients is undesirable in infants. Additionally, ingestion of preparations with high concentrations of all nutrients (e.g. in the form of pills or capsules) is not suitable, since this may result in hypervitaminosis. Providing these specific nutrients in adequate amounts on a weekly basis is sufficiently beneficial because of the buffering and storage capacity for these nutrients that is present in the body.

The present method comprises administering to the infant different types of meals, including a meal rich in iron, a meal rich in LC-PUFA, a meal rich in dietary fibre and preferably, also including a meal rich in zinc. Preferably at least one meal is consumed per day. More preferably one meal is consumed per day. The different types of meals are preferably consumed within a time period of 5-10 days, more preferably 7 days.

Furthermore, the present invention provides a kit of parts containing different nutritional compositions, suitable for use in the present method. The present kit has the advantage that parents do not need to keep detailed records of the nutritional components that the infant has already ingested during the week. The kit provides a tool for the parents to improve nutrition. In practise the intake of nutrients by the infant is often neglected, potentially resulting in deficiency of nutritional components. The present method and kit thus increase convenience and reduce the occurrence of nutrient deficiencies.

### DETAILED DESCRIPTION OF THE INVENTION

The present invention provides a method for providing nutrition to a human subject said method comprising administering to said human subject within a time period of 5 to 10 days;
a nutritional composition a.) comprising 0.35 to 6 mg iron per 100 g;
a nutritional composition b.) comprising 1.0 to 10 g fibre per 100 g; and
a nutritional composition c.) comprising 10 to 5000 mg long chain poly unsaturated fatty acids per 100 g;
and optionally a nutritional composition d.) comprising 0.15 to 4 mg zinc per 100 g; wherein compositions a., b. c. and d. comprise at least 1.5 g protein per 100 g, at least 5 g digestible carbohydrates per 100 g and at least 1.5 g fat per 100 g.

In other words the present invention provides the use of
a nutritional composition a.) comprising 0.35 to 6 mg iron per 100 g;
a nutritional composition b.) comprising 1.0 to 10 g fibre per 100 g; and
a nutritional composition c.) comprising 10 to 5000 mg long chain poly unsaturated fatty acids per 100g;
and optionally a nutritional composition d.) comprising 0.15 to 4 mg zinc per 100 g; wherein compositions a., b. c. and d. comprise at least 1.5 g protein per 100 g, at least 5 g digestible carbohydrates per 100 g and at least 1.5 g fat per 100 g,
for the preparation of a combination of compositions a., b., c. and optionally d. for providing nutrition to a human subject wherein said combination of compositions is to be administered to said human subject within a time period of 5 to 10 days. The combination of compositions can also be referred to as kit of parts. Thus in other words the present invention provides the use of a kit of parts as described herein in a method for providing nutrition to a human subject said method comprising administering to said human subject within a time period of 5 to 10 days the content of the kit of parts.

Preferably, the human subject is an infant.

In a further aspect, the present invention provides a kit of parts comprising 3 to 14 packaged nutritional compositions and including nutritional compositions a.), b.), c) and optionally d.), which comprise at least 1.5 g protein per 100 g, at least 5 g digestible carbohydrates per 100 g, and at least 1.5 g fat per 100 g, wherein
nutritional composition a.) comprises 0.35 to 6 mg iron per 100 g,
nutritional composition b.) comprises 1.0 to 10 g dietary fibre per 100 g; and,
nutritional composition c.) comprises 10 to 5000 mg long chain poly unsaturated fatty acids per 100g;
and optional nutritional composition d.) comprises 0.15 to 4 mg zinc per 100 g.

In a preferred embodiment nutritional composition a. comprises at least 0.7 mg iron per 100g, nutritional composition b. comprises at least 3.0 g fibre per 100 g, nutritional composition c., comprises at least 150 mg LC-PUFA per 100 g composition and, optional nutritional composition d. comprises at least 0.50 mg zinc per 100 g. Nutritional compositions containing these higher concentrations of iron, fibre, LC-PUFA and, optionally, zinc are especially suitable for infants recovering from disease.

The term "infants" as used in the present invention refers to human children aged from 4 months to 3 years. The present method is particularly suitable for infants aged from 6 to 36 months. Infants aged 11 up to 36 months are sometimes also referred to as "toddlers. In an especially preferred embodiment the present invention is intended for infants aged from 6 to 18 months.

### Method for feeding

The present invention provides a method for providing nutrition to a human subject, said method comprising administering to said human subject within a time period of 5 to 10 days, at least one nutritional composition rich in iron (nutritional composition a.), at least one nutritional composition rich in dietary fibre (nutritional composition b.), at least one nutritional composition rich in long chain polyunsaturated fatty acids (LC-PUFA) (nutritional composition c.), and preferably also including at least one nutritional composition rich in zinc (nutritional composition d.). In a preferred embodiment, the human subject is an infant.

In order to provide sufficient calories and macronutrients, the nutritional compositions a-d comprise proteins, digestible carbohydrates and fat, preferably at least 1.5 g protein per 100 g, at least 5 g digestible carbohydrates per 100 g, and at least 1.5 g fat per 100 g. Preferably, the nutritional compositions a., b., and c. and d. have a caloric density between 40 and 100 kcal per 100 gram, more preferably between 60 and 90 kcal per 100 g. Preferably, each of the present nutritional compositions provides between 50 and 300 kcal, more preferably between 100 and 300 kcal per serving.

Whenever reference is made to a weight of a nutritional composition (e.g. per 100 g, see above), this relates to the total weight of the nutritional composition as administered, including water.

Proteins, carbohydrate and fat are essential for growth of the infant. A balanced intake of each of these macronutrients contributes to a proper growth and development. Hence, preferably the nutritional compositions comprise at least 2 g protein per 100 g, more preferably at least 3 g of protein per 100 g. Preferably, the nutritional compositions contain less than 20 g protein per 100 g, more preferably less than 15 g protein per 100 g, even more preferably less than 10 g protein per 100 g.

Preferably the nutritional compositions comprise at least 6 g digestible carbohydrate per 100 g, more preferably at least 8 g of digestible carbohydrate per 100 g. Preferably, the nutritional compositions contain less than 40 g digestible carbohydrate per 100 g, more preferably less than 30 g digestible carbohydrate per 100 g, most preferably less than 20 g digestible carbohydrate per 100 g. Pr eferably the nutritional compositions contain potatoes, rice, and/or pasta. The term "digestible carbohydrate" as used herein refers to a carbohydrate capable of being converted into units that can be absorbed in mouth, esophagus, stomach and/or small intestine of the human alimentary canal.

Preferably the nutritional compositions comprise at least 2 g fat per 100 g, more preferably at least 3 g of fat per 100 g. The total amount of fat in the nutritional composition is preferably less than 20 g per 100 g, more preferably less than 10 g fat per 100 g, even more preferably less than 9 g fat per 100 g, most preferably less than 7 g fat per 100 g. Preferably, the fat component comprises essential fatty acids (EFA). EFA are fatty acids that cannot be manufactured by the body and fulfil important functions not served by alternative sources. There are two key families of EFA's, the omega-3 and the omega-6 family. Member of the omega-3 family of EFA's is alpha-linolenic acid (ALA, a fatty acid with a chain length of 18 carbon atoms and containing three double bonds). ALA is found in flaxseed and various vegetable oils and nuts. Member of the omega-6 family of EFA is linoleic acid (LA, a fatty acid with a chain length of 18 carbon atoms and containing two double bonds). The weight ratio omega-6/omega-3 fatty acids is preferably between 5 and 15. The fat component preferably comprises 0.3 to 1.5 g LA per 100, and at least 50 mg, more preferably at least 200 mg ALA per 100 g total fatty acid. The fat fraction preferably comprises below 58 wt.% saturated fatty acids based on total fatty acids, more preferably below 45 wt.%, most preferably below 35 wt.%. Preferably, the amount of saturated fatty acids is at least 5 wt.% of total fatty acids. The concentration of monounsaturated fatty acids is preferably between 17 and 60 wt.% based on total fatty acids. The concentration of polyunsaturated fatty acids is preferably between 11 and 36 wt.% based on total fatty acids.

Preferably, the present nutritional compositions contain between 75 and 90 wt. % water based on the total weight of the nutritional composition, more preferably between 78 and 85 wt. %.

In a preferred embodiment, the nutritional compositions to be administered to the infants each have a volume between 90 and 500 ml, more preferably between 125 and 300 ml. Preferably, the nutritional compositions used in the present method all have about the same volume (i.e. difference between greatest and smallest volume less than 50 ml).

In a preferred embodiment the meals are dinners. Dinners are the main meal of the day and can be served in the evening or at midday. Preferably, the meals are consumed at a temperature between 30 and 50, more preferably between 35 and 45 °C.

### Meal rich in iron (nutritional composition a.)

Nutritional composition a. contains at least 0.35 mg iron per 100 g, preferably at least 0.45 mg iron per 100 g, more preferably at least 0.7 mg iron per 100 g, most preferably at least 1.0 mg iron per 100 g meal. Nutritional composition a. preferably contains less than 6 mg iron per 100 g, preferably less than 5 mg iron per 100, more preferably less than 4 mg iron per 100 g. With iron is meant the sum of elemental iron, Fe²⁺ and Fe³⁺. Nutritional composition a. preferably comprises meat from a mammal, preferably at least one meat selected from the group consisting of beef, veal meat, pork meat, and lamb meat. Nutritional composition a. is preferably enriched in iron, e.g. supplemented by iron salts. Hence, in a preferred embodiment, nutritional composition a. comprises an iron salt selected from the group consisting of ferrous citrate, ferric ammonium citrate, ferrous gluconate, ferrous lactate, ferrous sulphate, ferrous fumarate, ferric diphosphate, ferric pyrophosphate, elemental iron, ferric saccharate, sodium ferric diphosphate, ferric casein, ferrous casein and ferrous carbonate. Preferably, the food ingredients of the meal, preferably vegetables and/or meat, are chosen in such a way that they provide amounts of iron described above. Preferably, composition b., c., and d. comprise less iron per 100 g composition than composition a.

### Meals rich in dietary fibre (nutritional composition b.).

The term dietary fibre as used herein refer to carbohydrates that enter the human colon intact after oral ingestion, i.e. which are not digested in the upper part of the gastrointestinal tract by enzymes or acid. Preferably nutritional composition b. contains fermentable dietary fibres, more preferably a water soluble fermentable dietary fibre. The term "fermentable" as used herein refers to the capability to undergo anaerobic breakdown and/or conversion by micro-organisms normally present in the lower part of the human gastro-intestinal tract (e.g. colon) to smaller molecules, in particular short chain fatty acids and lactate. The fermentability may be determined by the method described in Am. J. Clin. Nutr. 53, 1418-1424 (1991).

Nutritional composition b. preferably contains at least 1.0 g dietary fibre per 100 g meal, preferably at least 1.5 g dietary fibre per 100 g, more preferably at least 2.5 g dietary fibre per 100 g, most preferably at least 3.0 g dietary fibre per 100 g. Nutritional composition b. preferably contains less than 10 g dietary fibre per 100 g, preferably less than 7 g dietary fibre per 100 g, more preferably less than 5 g dietary fibre per 100 g. Fibres are suitably quantitated by AOAC method 985.29., AOAC method 2000.11., the method described by de Slegte, J. 2002, Journal of AOAC International 85, 417-23., and AOAC method 999.03.

Preferably, nutritional composition b. comprises at least three fibres selected from the group consisting of soy fibre, cellulose, resistant starch, gum acacia, pectin, inulin, and hydrolysed inulin. Preferably, nutritional composition b. comprises at least one non-digestible, fermentable dietary fibre selected from the group consisting of polyfructose, fructo-oligosaccharides, galacto-oligosaccharides, partially hydrolysed galactomannan, acidic oligosaccharides and indigestible polydextrin, more preferably at least galactooligosaccharides. In an especially preferred embodiment composition b. comprises a) a mixture of transgalactooligosaccharides and polyfructose; or b) a mixture of partially hydrolysed guar gum with one carbohydrate selected from the group consisting of polyfructose and resistant or indigestible polydextrin.

Polyfructose is a polysaccharide carbohydrate comprising a chain of β-linked fructose units with a degree of polymerisation (DP) of 10 or more fructose units, e.g. inulin. Fructo-oligosaccharides (FOS) are glucose- and/or fructose-terminated fructose chains, with a DP below 10. Thus, FOS can be described as GFₙ₋₁ chains or Fₙ chains, wherein G is a glucosyl unit, F is fructosyl unit and n = 1-9.

Galactooligosaccharides (GOS) are oligosaccharides comprising galactose units, with a DP of less than 10. A glucose unit may be present at the reducing end of the chain. GOS comprises α- and β-galacto-oligosaccharides. Preferably at least 66 % of the saccharides units of the GOS are galactose units. Trans-galacto-oligosaccharides (TOS) are galacto-oligosaccharides in which the majority of the galactose units (at least 90 %, preferably at least 95 %) are linked by β-bond, for example β-(1,4 bonds). Preferably, at least 50 % of the bonds of the GOS as used in the present invention are β-bond. Such a TOS is for example that found in Vivinal®GOS (Borculo Domo Ingredients, Zwolle, Netherlands).

The term acid oligosaccharide refers to oligosaccharides comprising at least one acidic group selected from the group consisting of N-acetylneuraminic acid, N-glycoloylneuraminic acid, free or esterified carboxylic acid, sulfuric acid group and phosphoric acid group. The acidic oligosaccharide preferably comprises uronic acid units (i.e. uronic acid polymer), more preferably galacturonic acid units. The acid oligosaccharide may be a homogeneous or heterogeneous carbohydrate. Preferably hydrolysates of pectin and/or alginate are used as acidic oligosaccharides. The DP is preferably below 10.

Partially hydrolysed galactomannan refers to a composition in which galactomannan has been subjected to hydrolyses and has not been hydrolysed to its monomeric units. Galactomannan are polysaccharides comprising at least 90 %, preferably at least 95 %, of β-(1,4)-D-mannopyrasyl units in the linear chain, and galactose branches bound thereto via α-(1,4)-D bonds. According to a particularly preferred embodiment guar gum is used.

Indigestible polydextrin, refers to indigestible carbohydrates which have a DP of 3 to 50, preferably of 4 to 20 and in which the monomeric units are at least 80 %, preferably at least 85 % originating from glucose (based on the total of monomeric units present). The average degree of polymerisation is between 10 and 16 monosaccharide units per molecule. In a preferred embodiment, the indigestible polydextrins are randomly branched and comprise α-(1,4), α-(1,6) glucosidic bonds and α/β-(1,2), α/β-(1,3),and β-(1,6) linkages. Indigestible polydextrins are for example available under the tradename "Fibersol 2®" from Matsutami Inductries or Litesse® from Danisco.

Preferably, nutritional composition b. does not contain meat, fish or poultry. Preferably, composition b. comprises egg, cheese, mushrooms, texturised vegetable proteins, fermented soy beans, soy protein concentrates and/or legumes. Preferably, the natural ingredients of the meal, such as the vegetables, potatoes, rice, pasta, mushrooms, and/or legumes, provide the amounts of fibre as described above. Preferably, composition a., c., and d., comprise less fibre per 100 g than composition b.

### Meals rich in LC-PUFA (nutritional composition c.)

Long chain polyunsaturated fatty acids (LC-PUFA) are fatty acids containing at least two double bonds and a carbon chain length of 20-22 carbons. The term LC-PUFA includes eicosapentaenoic acid (EPA), docosahexaenoic acid (DHA) and arachidonic acid (ARA). In a preferred embodiment this meal is rich in omega-3 LC-PUFA's, since in the Western diet the balance of ratio of omega-6 to omega-3 fatty acids is too high, which is not optimal for disease prevention and health. Preferably, nutritional composition c. contains DHA and EPA.

Meals rich in LC-PUFA contain a source that is rich in LC-PUFA such as fish and/or marine vegetables, such as seaweed and/or algae. Preferably nutritional composition c. contains fish, more preferably salmon, cod, tuna, and/or fish oil.

Nutritional composition c. comprises at least 10 mg LC-PUFA per 100 g, preferably at least 40 mg LC-PUFA per 100 g, more preferably at least 100 mg LC-PUFA per 100 g, most preferably at least 150 mg LC-PUFA per 100 g. Nutritional composition c. preferably contains less than 5 g LC-PUFA per 100 g, preferably less than 3 g LC-PUFA per 100 g, more preferably less than 1 g LC-PUFA per 100 g.

Preferably, the natural ingredients of the meal, such as fish, are chosen in such a way that they provide the amounts of LC-PUFA as described above. Preferably, composition a., b., and d. comprise less LC-PUFA per 100 g than composition c.

In a preferred embodiment vitamin E is present to prevent oxidation of the polyunsaturated fatty acids. Preferably, at least 3 mg, more preferably at least 6 mg vitamin E per 100 g composition is present.

### Nutritional composition d.

In a preferred embodiment besides nutritional composition a., b., and c., also a fourth nutritional composition is present, nutritional composition d. Preferably, nutritional composition d. comprises a minimal level of nutrient selected from the group consisting of iodine, and zinc. Preferably, nutritional composition d. comprises a minimal level of zinc.

Iodine is an essential component of the thyroid hormones, and iodine deficiency is associated with hypothyroidism, with goitre, and growth and mental retardation. Such endemic iodine deficiency disorders have been shown to exist even now in European countries. Effects of fortification of salt and salt in bread may not be the optimal solution for toddlers, because of their low salt and bread intake. Meals rich in iodine contain a source that is rich in iodine such as seafood and vegetables cultured on iodine rich soil. The amount of iodine is at least 2 µg per 100 g. Preferably, the amount of iodine is at least 5 µg per 100 g. More preferably, the amount of iodine is at least 10 µg per 100 g. Most preferably, the amount of iodine is at least 12 µg per 100 g. The meal contains maximally 70 µg iodine per 100 g. Preferably the composition contains less than 60 µg iodine per 100 g. More preferably the composition contains less than 45 µg iodine per 100 g. Preferably, the natural ingredients of the meal are chosen in such a way that they provide sufficient amounts of iodine. Preferably, extra iodine may be added in the form of sodium iodide, potassium iodide, potassium iodate, sodium iodate and/or mixtures thereof.

### Meal rich in zinc (nutritional composition d.)

Nutritional composition d. comprises at least 0.15 mg zinc per 100 g, preferably at least 0.2 mg zinc per 100 g, more preferably at least 0.35 mg zinc per 100 g, most preferably at least 0.5 mg zinc per 100 g. Nutritional composition d. preferably comprises below 4 mg zinc per 100 g, preferably less than 3.5 mg zinc per 100 g, more preferably less than 2.5 mg zinc per 100 g.

Nutritional composition d. preferably comprises ingredients which are a good natural source of zinc, such as legumes, nuts and/or or the chicken, preferably the dark meat of chicken. Preferably, nutritional composition d. comprises poultry meat (e.g. turkey and/or chicken), to provide a variation in animal derived food sources. In a further preferred embodiment, nutritional composition d. contains at least one zinc source selected from the group consisting of zinc acetate, zinc citrate, zinc lactate, zinc sulphate, zinc oxide and zinc gluconate.

Preferably, the natural ingredients of the meal, such as legumes or chicken, are chosen in such a way that they provide the amounts of zinc as described above. Preferably, composition a., b., and c., comprise less zinc per 100 g than composition d.

### Kit of parts

The present invention relates to a method for providing a well balanced diet to infants. The meals according to the present invention are individually packaged and preferably provided as a kit of parts, wherein said kit of parts contains several different meals, i.e. meals with different nutritional compositions as described hereinabove.

The present kit comprises at least 3, more preferably 3 to 14, more preferably at least 4, even more preferably 4 to 11, and most preferably 7 individually packed nutritional compositions, which have a caloric content of between 40 and 100 kcal per 100 g. The kit preferably contains at least the nutritional composition a, b and c, more preferably at least nutritional compositions a., b., c. and d. described hereinabove. In an even further preferred embodiment, the present kit contains 2-4 packaged nutritional compositions a.; 1-2 packaged nutritional compositions b.; 1-2 packaged nutritional compositions c.; and 0-3 packaged nutritional compositions d.

The nutritional compositions in the kit are preferably in ready-to-eat and/or dried form. From the dried form, a ready-to-eat form can be easily produced by reconstitution in a suitable liquid, e.g. water. The ready-to-eat form can normally be administered directly to the infant, optionally after heating and/or mixing. The nutritional compositions in the kit preferably have a volume between 100 and 500 ml, more preferably between 150 ml and 300 ml.

The individually packaged nutritional compositions are preferably packaged in single holder-packaging, enabling the kit to be recognised as a single unit. Preferably the holder-packaging is a carton box. The holder packaging is preferably labelled with indications that the packed nutritional compositions contained therein should be administered within a 5 to 10 days period, preferably a 7 day period. Preferably the period in days indicated on the package corresponds with the quantity of individually packaged nutritional compositions with a caloric content between 100 and 300 kcal contained therein. Most preferably, on the holder packaging it is indicated that the kit contains 7 nutritional compositions, which are to be administered within a 7-day period.

In a further preferred embodiment, the nutritional compositions of the kit of parts are unified by that each of the individually packaged nutritional compositions contains labelling or indicia, said labelling or indicia specifying the other nutritional compositions of the kit, or alternatively, the said labelling on the individually packed nutritional compositions making reference to the present method described hereinabove.

Each individually packaged nutritional composition as present in the kit is preferably packed in a glass or plastic container with cap or lid. The individually packed nutritional composition preferably takes the form of a single container containing a single composition consisting of a mixture of ingredients, which are either completely or partially mixed. More preferably, at least one of the individually packed nutritional composition in the present kit is a plate containing separate compartments, containing different elements of the nutritional compositions (e.g. meat and vegetables separated), which together make up nutritional composition a., b., c., or d.

In order to enable easy recognition of the nutritional constituents within the containers providing one nutritional composition, the containers are preferably provided with indicia (e.g. words, colour, logo and/or number). The containers further contain coloured lids or caps.

Preferably, the nutritional composition a. is packed in a container with a red lid or cap; nutritional composition b. is packed in a container with a green lid or cap; nutritional composition c. is packed in a container with a blue lid or cap. Preferably, nutritional composition d. is packed in a container with a yellow lid or cap.

The containers preferably bear indicia, for example on the label, expressing that the content of the container should be preferably consumed in a particular frequency on a weekly basis. Preferably the content of a container is consumed in one meal.

The present invention also relates to an article of manufacture comprising a packaging comprising 3 to 14 nutritional compositions, comprising:
- nutritional composition a.) comprising at least 1.5 g protein per 100 g, at least 5 g digestible carbohydrate per 100g, at least 1.5 g fat per 100 g, and 0.35 to 6 mg of iron per 100 g,
- nutritional composition b.) comprising at least 1.5 g protein per 100 g, at least 5 g digestible carbohydrates per 100 g, at least 1.5 g fat per 100 g, and 1.0 to 10 g fibre per 100 g,
- nutritional composition c.) comprising at least 1.5 g protein per 100 g, at least 5 g digestible carbohydrates per 100 g, and at least 1.5 g fat per 100 g, and 10 to 5000 mg LC-PUFA per 100g.
- optionally nutritional composition d.) comprising at least 1.5 g protein per 100 g, at least 5 g digestible carbohydrate per 100 g, at least 1.5 g fat per 100 g, and 0.15 to 4 mg of zinc per 100 g,

When the meals are dried the weight of the composition will be reduced. All weights in the present invention refer to the weight of reconstituted or ready-to-eat meals. The meals may be stored frozen, cooled or be stable at ambient temperatures. Preferably the meals will be stable at ambient temperatures, i.e. the kit can be stored at room temperature.

### EXAMPLES

### Example 1:

Kit of parts containing nutritional compositions rich in fibre, LC-PUFA and iron. Kit of parts comprising 3 meals rich in iron (meat containing meal), 2 meals rich in LC-PUFA (fish containing meal) and 2 meals rich in fibre (vegetarian meal) for infants of 8 months and older. Each meal has a weight of 200 g.

Meat containing meal: Nutritional composition, packed in a glass jar with a red lid on which the logo of a cow is visible, which contains 35 wt. % mixed vegetables (14 wt. % carrot, 10 wt. % green beans, 6 wt.% white beans, 5 wt.% peas), 10 wt.% beef, 25 wt. % potato, 25 wt. % beef extract and 5 % others (wheat flour, corn oil, water). The composition contains 76 kcal per 100 g, 3.4 g protein per 100 g, 9.7 g. digestible carbohydrates per 100 g, and 2.8 g fat per 100 g. The composition contains 0.81 mg iron per 100 g. A label indicates to consume three of these meat containing meals per week. The label further indicates that it is recommended to consume on a weekly basis, and in addition to the meat containing meals, 2 meals containing fish (see below) and 2 vegetarian meals (see below).

Fish containing meal: Nutritional composition, packed in a glass jar with a blue lid on which the logo of a fish is visible, which contains 38 wt. % mixed vegetables (19 wt. % tomatoes, 19 wt. % carrots), 10 wt.% salmon, 19 wt. % rice, and 33 % others (wheat flour, corn oil, water, skimmed milk, onions, parley, lemon juice, thyme). This composition contains 77 kcal/100g. The composition contains 3.1 g protein per 100 g, 9.7 g digestible carbohydrates per 100 g, and 3.1 g fat per 100 g. The composition contains 190 mg LC-PUFA per 100 g. A label indicates to consume two of these fish containing meals per week. The label further indicates that it is recommended to consume on a weekly basis, and in addition to the fish containing meals, 3 meals containing meat (see above) and 2 vegetarian meals (see below).

Vegetarian meal (fibre): Nutritional composition, packed in a glass jar with a green lid on which the logo of a leaf is visible, which contains, 27 wt. % tomatoes, 5 wt. % mushrooms, 5 wt. % onion, 2 wt. % Corn, 22 wt. % brown beans and 21 wt. % rice. 18 wt. % others (corn starch, vegetable oil, yeast extract, pine apple juice, water). The composition contains 75 kcal per 100 g. The composition contains 2.8 g protein per 100 g, 13.0 g digestible carbohydrate per 100 g, and 1.3 g fat per 100g. The composition contains 3.22 g dietary fibre per 100 g. A label indicates to consume two of these fibre rich meals per week. The label further indicates that it is recommended to consume on a weekly basis, and in addition to the fibre rich, 2 meals containing fish (see above) and 2 vegetarian meals (see above).

### Example 2:

Kit of parts containing nutritional compositions rich in fibre, LC-PUFA, iron and zinc. Kit according to example 1, containing in addition, 2 poultry containing meals. The kit contains one instead of two vegetable meals and fish containing meals. References to the other meals are adapted accordingly.

Poultry containing meal: Nutritional composition packed in a glass jar with a yellow lid on which the logo of a chicken is visible, which contains 35 wt. % mixed vegetables (40 wt. % carrot, 3.5 wt. % white beans), 10 wt.% chicken, 29 wt. % rice, and 18 % others (corn oil, water). The composition contains 73 kcal per 110g. The composition contains, 2.9 g protein per 100 g, 8.5 g digestible carbohydrates per 100 g and 3.2 g fat per 100 g. The composition contains 0.64 mg zinc per 100 g. A label indicates to consume two of these poultry containing meals per week. The label further indicates that it is recommended to consume on a weekly basis, and in addition to the poultry containing meal, 3 meals containing meat (see above), 1 meals containing fish (see above) and 1 vegetarian meal (see above).

### Example 3:

Kit of parts containing nutritional compositions rich in fibre, LC-PUFA, iron and zinc. Individually packaged nutritional compositions (in total 7) as described in Example 2, with or without the specified labeling, packaged in a carton box.

### Example 4:

Kit of parts containing nutritional compositions rich in fibre, LC-PUFA, iron and zinc, according to example 2 or 3, wherein the meals are supplemented in order to obtain high concentrations of specific nutrients.

Meat containing meal comprises 1.5 mg iron per 100 g obtained by supplementing meat containing according to example 1 with ferrous lactate.

Vegetarian meal comprises 4 g dietary fibre per 100 g obtained by supplementing fibre containing composition of example 1 with a mixture of transgalactooligosaccharides and inulin.; and

Fish containing meal comprises 250 mg long chain poly unsaturated fatty acids per 100 g, obtained by supplementing fish containing composition of example 1 with salmon oil.

## Claims

1. Kit of parts comprising 3 to 14 packaged nutritional compositions and including nutritional compositions a.), b.), and c.), which comprise at least 1.5 g protein per 100 g, at least 5 g digestible carbohydrates per 100 g, and at least 1.5 g fat per 100 g, wherein
nutritional composition a.) comprises 0.35 to 6 mg iron per 100 g,
nutritional composition b.) comprises 1.0 to 10 g dietary fibre per 100 g; and,
nutritional composition c.) comprises 10 to 5000 mg long chain poly unsaturated fatty acids per 100 g.

2. Kit of parts according to claim 1, further comprising nutritional composition d.), containing at least 1.5 g protein per 100 g, at least 5 g digestible carbohydrates per 100 g, at least 1.5 g fat per 100 g, and 0.15 to 4 mg zinc per 100 g.

3. Kit of parts according to claim 1 or 2, wherein the kit of parts consists of 3 to 14 packaged nutritional compositions.

4. Kit of parts according to any one of the preceding claims, comprising between 4 and
11 packaged compositions, consisting of
2 to 4 nutritional compositions a.;
1 or 2 nutritional compositions b.;
1 or 2 nutritional compositions c.;
0 to 3 nutritional compositions d.

5. Kit of parts according to any one of the preceding claims wherein nutritional composition b.) comprises at least one fibre selected from the group consisting of polyfructose, fructo-oligosaccharides, galacto-oligosaccharides, partially hydrolysed galactomannan, acidic oligosaccharides and indigestible polydextrin.

6. Kit of parts according to anyone of the preceding claims, wherein the nutritional compositions a., b., c., and optionally d., have a caloric density between 40 and 100 kcal per 100 gram.

7. Kit of parts according to anyone of the preceding claims for use in a method for providing nutrition to a human subject.

8. Method for providing nutrition to a human subject said method comprising administering to said human subject within a time period of 5 to 10 days;
a nutritional composition a.) comprising 0.35 to 6 mg iron per 100 g;
a nutritional composition b.) comprising 1.0 to 10 g fibre per 100 g; and
a nutritional composition c.) comprising 10 to 5000 mg long chain poly unsaturated fatty acids per 100g;
wherein compositions a., b. and c. comprise at least 1.5 g protein per 100 g, at least 5 g digestible carbohydrates per 100 g and at least 1.5 g fat per 100 g.

9. A method according to claim 7 or 8 in which the human subject is an infant.

10. Method according to claim 8 or 9, further comprising administering, within the time period, nutritional composition d.) comprising at least 1.5 g protein per 100 g, at least 5 g digestible carbohydrates per 100 g, at least 1.5 g fat per 100 g, and 0.15 to 4 mg zinc per 100 g.

11. Method according to any one of claim 8 to10, wherein compositions a., b. c. and optionally d. are administered to the infant on separate days.

12. Method according to any one of claims 8 to 11 comprising administering to the human subject with the time period: 2 to 4 times nutritional composition a.), 1 to 2 times nutritional composition b.), 1 or 2 times nutritional composition c.), and 0 to 3 times nutritional composition d.).

13. Kit of parts according to any one of the claims 1-6 adapted for carrying out the method of any one of the claims 8-12.
